# EUROPEAN PATENT SPECIFICATION

(11) **EP 3 064 911 B1**
(45) Date of publication and mention of the grant of the patent: **19.07.2023**
(21) Application number: 15461511.6
(22) Date of filing: 02.03.2015
(51) Int. Cl.: G01L 1/20, G01L 1/26, A61B 5/11, A61B 5/00, B25F 5/00, G01H 1/00, G01H 11/00

(54) **AN INTEGRATED VIBRATIONS AND CONTACT FORCE CONVERTER AND A METHOD FOR MEASURING VIBRATIONS AND CONTACT FORCE**
INTEGRIERTER SCHWINGUNGS- UND KONTAKTKRAFTWANDLER UND VERFAHREN ZUR MESSUNG DER SCHWINGUNGS- UND KONTAKTKRAFT
CONVERTISSEUR INTÉGRÉ DE VIBRATIONS ET DE FORCE DE CONTACT ET PROCÉDÉ DE MESURE DE VIBRATIONS ET DE FORCE DE CONTACT

(43) Date of publication of application: 07.09.2016
(73) Proprietor: SVANTEK Sp. z o.o., 04-872 Warszawa (PL)
(72) Inventor: Barwicz, Wieslaw, 01-651 Warszawa (PL); Kosowski, Artur, 05-400 Otwock (PL); Gorski, Slawomir, 03-134 Warszawa (PL); Kaminski, Witold, 03-952 Warszawa (PL)
(74) Representative: Kancelaria Eupatent.pl Sp. z.o.o

(56) References cited:
- EP-A1- 1 586 875
- EP-A2- 2 003 429
- WO-A2-2010/041059
- DD-A1- 247 308
- DE-A1- 10 119 252
- GB-A- 2 495 559
- US-A1- 2003 163 287

## Description

### TECHNICAL FIELD

The present invention relates to measurement of vibrations and contact force acting on human arms, in particular vibrations occurring during manual operation of tools.

### BACKGROUND

There are various tools that, when operated, generate and transfer vibrations to arms of an operator. Such tools include devices for fractioning, separating, tightening, milling, crushing, drilling, boring or polishing. Many of these devices are operated manually, therefore the vibrations generated by the device are input to the human body, which may negatively impact human's health or even lead to long-term illnesses. Operators of vibrating devices are subject to hand-arm vibration disease syndrome, resulting primarily in secondary Raynaud's phenomenon causing pale fingers and leading to irreversible disruptions in functionality of the organism. It is therefore advisable to monitor the exposure of the operator to vibrations and to stop work when the exposure exceeds safeguard values.

There are known vibration metering devices, so-called vibrometers. There are known various standards for measuring vibrations, specifying methods of measurement and allowable average and maximum values to which the operator can be exposed.

However, the solutions known so far do not allow precise measurements of vibrations to be performed directly on the palm of hand of the operator, to determine the exact vibrations to which the operator is subjected. Another problem that occurs during measurement of vibrations is that not only the amplitude of vibrations at the operator's hand should be measured, but also the force with which the operator pushes or presses the device. Only by this manner the dose of vibration which are input to the organism can be measured.

For example, an operator of a device generating vibrations of a low amplitude, who presses the device using large force, may be subject to a higher health risk than an operator of a device generating vibrations of a high amplitude, who only gently touches that device does not contact it continuously. It is essential to conduct measurement of a stream of energy of vibrations entering to the organism of the operator - this requires information about the contact force between the operator's hand and the device. Such measurements of vibrations amplitude and contact force were conducted so far only in laboratories.

A European patent application EP2003429 discloses a monitoring device comprising a vibration sensor, a mounting and a control device, the vibration sensor positioned in use upon vibrating equipment using the mounting to provide a vibration characteristic for the equipment in use.

A German patent application DE10119252 discloses a measurement transducer for hand-arm vibrations and a measurement transducer for whole body vibrations, but does not describe its constructional features in details.

There is a need to provide solution for measuring vibrations that would allow performing the above mentioned measurements under working conditions.

### SUMMARY

The object of the invention is an integrated vibrations and contact force converter, as well as a method for measuring vibrations and contact force transmitted from a vibrating tool to operator's body, according to the appended claims.

### BRIEF DESCRIPTION OF FIGURES

The invention is shown by means of example embodiment on a drawing, in which:
Fig. 1A shows an integrated converter in a general view with a strip for attaching it to a hand;
Fig. 1B shows the integrated converter according to the first embodiment in a bottom view;
Fig. 1C shows the integrated converter according to the first embodiment in a cross-sectional view along the line A-A of Fig. 1B;
Fig. 2 shows an exploded view of the embodiment of the integrated converter according to the first embodiment;

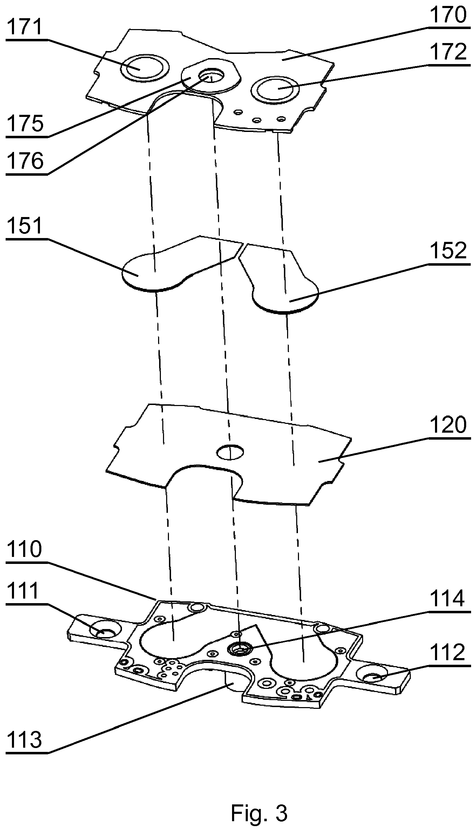

Fig. 3 shows an exploded view of a measuring module for the first embodiment in an inverted view from the bottom to the top;
Fig. 4 shows the enlarged detail B of the cross-section shown in Fig. 1C;
Fig. 5 shows a simplified functional schematic of the converter according to the first embodiment;
Figs. 6A shows the integrated converter according to a second embodiment in a bottom view;
Fig. 6B shows the integrated converter according to the second embodiment in a cross-sectional view along the line A-A of Fig. 1A;
Fig. 7 shows an exploded view of the embodiment of the integrated converter according to the second embodiment;
Fig. 8 shows an exploded view of a measuring module for the second embodiment in an inverted view from the bottom to the top;
Fig. 9 shows the enlarged detail B of the cross-section shown in Fig. 6B;
Fig. 10 shows a simplified functional schematic of the converter according to the second embodiment;
Fig. 11A-11C show further embodiments of the casing of the converter.

### DETAILED DESCRIPTION

In the first embodiment presented herein, the integrated tri-axial converter of acceleration of vibrations and contact force to an electric signal comprises a casing comprising a top part 11 and a bottom part 12, between which a measurement module 20 is mounted. The measurement module 20 is connected with the top part 11 of the casing by side screws 13, 14 and a central screw 15, such as to provide a rigid connection between the measurement module 20 and the top part 11 of the casing. The bottom part 12 of the casing 10 is connected with the measurement module 20 by means of screw 16 having controlled pressure force, by means of which a minimum pressure force is achieved that guarantees correct measurement of acceleration of vibrations, and which allows for further pressure of the bottom part 12 of the casing 10 with respect to the measurement module 20 and the top part 11 of the casing 10 during vibrations measurement, which allows measurement of contact force.

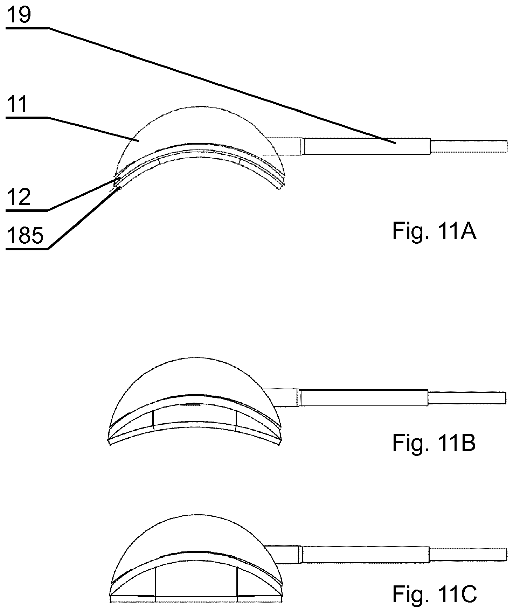

The casing 10, comprising a top part 11 (considered as the portion for contacting the operator's hand) and a bottom part 12 (considered as the portion for contacting the tool), has a body which is shaped depending on the shape of the tool which is to be subject to measurement, as shown by the examples of figs. 11A-11C. The top part 11 of the casing 10 has an oblique and convex shape, such as to fit comfortably into the internal part of operator's palm. The shape of this casing part is preferably corresponding to the requirements of ISO 10819 standard (related to laboratory measurements of anti-vibration gloves).

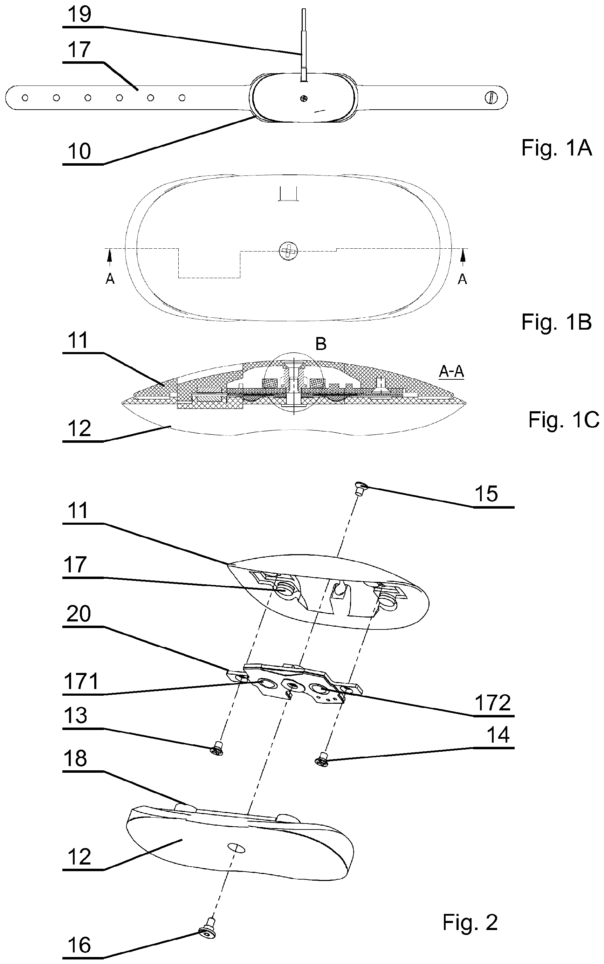

The bottom part 12 of the casing 10 is replaceable, and therefore it can be selected depending on the tool used. This increases the versatility of the converter, as the main measurement module 20 together with the top casing 11 forms the main part of the device, and only the bottom parts 12 are replaceable. The shape of the bottom part 12 can be concave, such that it fits as precisely as possible to the handle of a vibrating tool in the form having an oval cross-section. The integrated converter is designed to be worn during use at the internal side of the palm, it can be attached to the hand by means of a strap (e.g. strap 17 as shown in Fig. 1A) to be wound around the hand. The integrated converter can be also used inside the working glove.

The measurement module 20 comprises the following elements. The main module 110 is a printed circuit board (PCB), on which there are mounted various electronic circuits for measuring vibrations, a 3-axis acceleration sensor, a signal concentrator, a processor or a data transmission circuit. Moreover, the PCB comprises signal paths and power supply paths. The main module 110 has openings 111, 112 and a connector 113 for connecting the module in a rigid manner with the top part 11 of the casing via screws 13, 14, 15. Preferably, the whole volume between the measurement module 20 and the top part 11 of the housing is filled with a stiffening agent, preferably epoxy resin, in order to eliminate the self-resonance of the board in the useful measurement band. The circuit of the PCB is connected with contact-sensitive elements, i.e. force sensors 151, 152, such as force sensitive resistors (FSRs). Another element is a rubber spacer 170 having at least 80 Shore A hardness. The spacer 170 has formed hemispherical protrusions 171, 172, which together with a rubber collar 175 form a force divider, as a portion of the contact force is applied to the collar 175. The collar 175 has an opening 176 for a screw 16 connecting the bottom part 12 of the casing 10 with the opening 114 in the main module 110.

Moreover, the top part 11 of the casing comprises magnetic or metal elements 17 cooperating with respective metal or magnetic elements 18 in the bottom part 12 of the casing, which attract each other and facilitate preliminary assembly of the casing before fastening the screw 16.

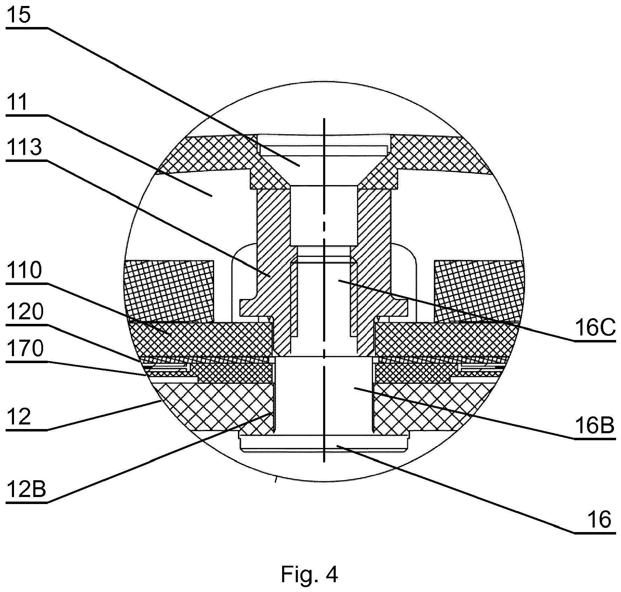

As shown in details in Fig. 4, the bottom part 12 of the casing is screwed by the screw 16 to the connector 113 of the measurement module 20 such as to impress an initial force (an offset) on the force sensors 151, 152. The top part 11 of the casing is screwed by the screw 15 to the connector 113, such that it is rigidly fixed to the measurement module 20. The screw 16 has a distancing portion 16B having a diameter smaller than the opening 12B in the bottom part of the casing than the opening 176 of the elastic spacer 170, and a threaded portion 16C for connecting the screw 16 with a threaded hole 114 in the main module 110. Therefore, the bottom part 12 of the casing is movable with respect to the top part 11 of the casing 10.

The initial pressing exerted by screwing the screw 16 shall be relatively small with respect to the total measurement range of the sensors 151, 152, and at the same time sufficiently large so as to allow appropriate transfer of vibrations within the required frequency band (2kHz). After the device is mounted, calibration of the force measurement path is performed. The initial setting causes pre-stress of the force sensors 151, 152 and increase of the pressing does not affect transmission of vibrations in the useful band, but only a change in the force exerted on the resistors 151, 152.

In order to provide adequate parameters of vibrations transmission, the elastic spacer 170 shall have at least 80 Shore A hardness. Then, the elastic spacer 170 provides adequate coupling between the bottom part and the top part, which enables desired frequency characteristic of the measurement of acceleration of vibrations. The coupling force of the bottom part and the top part must be within appropriate range, between a minimum and maximum value (which would limit the top range of the force measurement) - the device may signal an error, if the bottom part is fastened inappropriately (i.e. too lose or too tight) to the top part.

The mechanical filter, responsible for dampening the signal of acceleration of vibrations having frequencies above the useful band, is formed mainly by the layer of rubber 18 adhered to the bottom part of the casing.

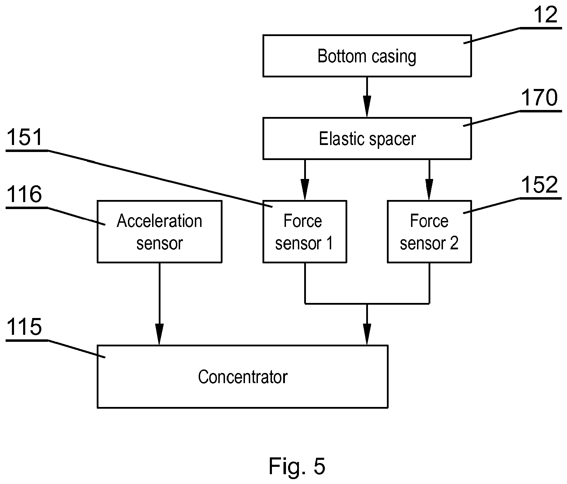

Fig. 5 shows a simplified functional schematic of the integrated converter for the first embodiment. The integrated converter comprises a concentrator 115 of signals from the acceleration and force sensors. The concentrator 115 can be a circuit for transmitting signals from the sensors to other circuits or to external devices, or a processor for gathering and processing data from sensors and transmitting the data through a wire 19. The concentrator 115 receives signal from the acceleration sensor 116 mounted together with the concentrator 115 on the PCB of the main module 110. The acceleration sensor 116 can be a 3-axis MEMS sensor. Moreover, force sensors 151, 152 are connected to the concentrator 115 via adequate amplifiers. Preferably, the sensors 151, 152 are connected in parallel. During operation of the system, the acceleration sensor 116 provides information related to amplitude of vibrations and the force sensors 151, 152 provide information related to the force by which the vibrations act on the bottom part 12 of the casing, and thereby to the body of the operator.

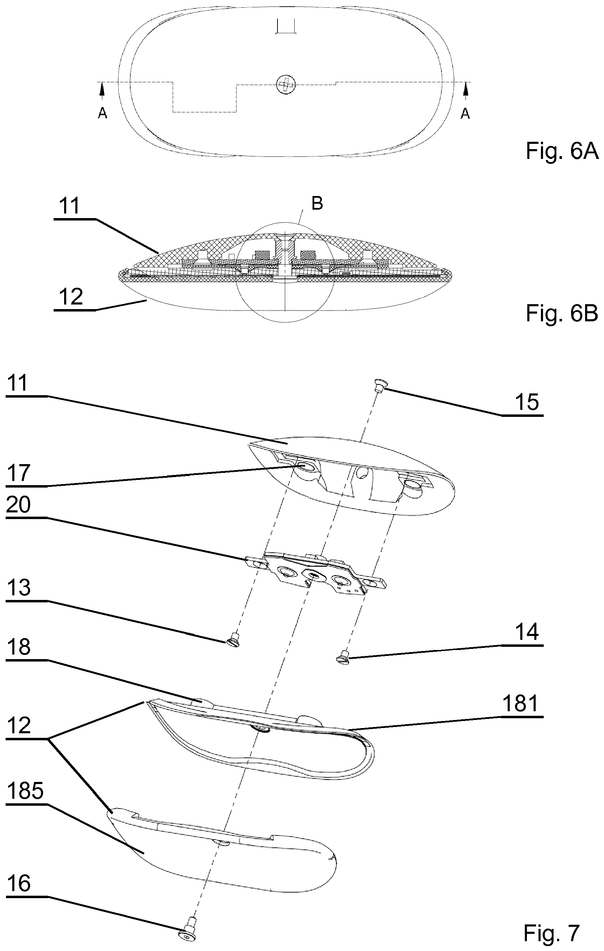

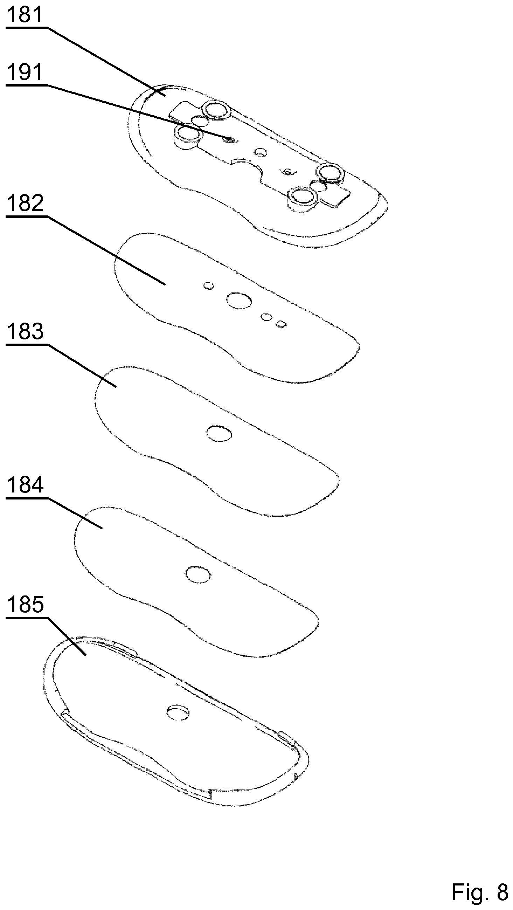

Figs. 6-10 show a second embodiment of the converter. The converter according to the second embodiment may have a configuration such as shown in Fig. 1A and Fig. 11A-11C. The second embodiment differs from the first embodiment described above mainly in that it comprises a force sensor integrated with the bottom part 12 of the casing and fixed (preferably, glued) to substantially the whole area of the bottom part 12 of the casing. The exploded view of the bottom part 12 of the casing according to the second embodiment is shown in Fig. 8. The bottom part of the casing comprises a bottom cover 181, to which an elastic printed circuit board (PCB) 182 is attached. The elastic PCB 182 comprises paths which divide the contact area to parallel segments for measuring the area resistance, for example in form of a comb. Moreover, the PCT 182 may comprise miniature electronic circuits, such as a TEDS memory for storing data related to the sensitivity of the force-sensitive layer 183 and transferring these data to the concentrator 115. Another element is a force-sensitive layer 183 in form of a force-sensitive resistor (FSR). For example, the layer 183 may be formed from polymer thick film (PTF), whose resistance decreases as the contact force increases. The resistor 183 can be connected to a separating layer 184, preferably made of a plastic foil, e.g. from polyethylene, used to protect the resistor 183 from damage by a rubber cover 185 moving under vibrations of the tool during its operation. The rubber band 185 is positioned at the bottom of the device, i.e. at the side for contacting the tool, and it has preferably high hardness, comparable to the hardness of the internal rubber spacer, preferably at least 80 Shore A hardness, preferably 90 Shore A hardness. The layer 185 transfers vibrations to the foil 184 and to the resistor 183, facilitates keeping the converter on the tool and filters high frequency vibrations, i.e. vibrations above 2kHz.

The construction of the second embodiment allows to set a calibration coefficient to each bottom part independently via the TEDS memory integrated with it. This allows auto-calibration of the converter without engagement of the user.

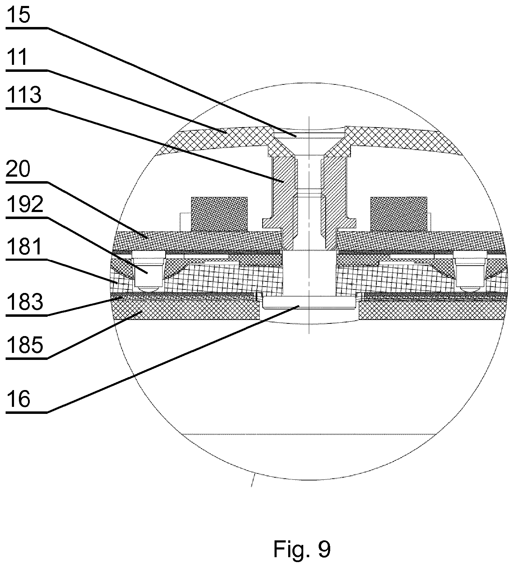

As shown in Fig. 9, the bottom part 12 of the casing, formed by integrated layers 181-185, is fastened by the screw 16 to the connector 113 of the measurement module 20, such as to exert initial pressure on the force-sensitive layer 183 via resilient contact elements 192 passing through openings 191 in the bottom cover 181. The resilient contact elements 192 conduct a signal informing about the contact force from the force-sensitive layer via the elastic PCB 182 to the measurement module 20. The top part 11 of the cover is fastened by the screw 16 to the connector 113, such that it is fixed still to the measurement module 20.

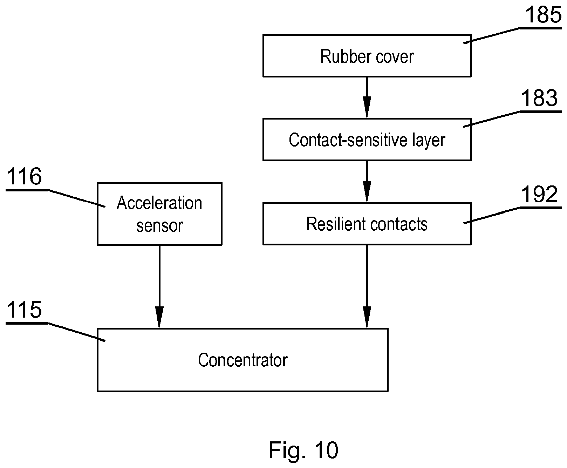

Fig. 10 shows a simplified functional schematic of the integrated converted for the second embodiment. The integrated converter comprises a concentrator 115 of signals from the acceleration and force sensors. The concentrator 115 can be a circuit for transmitting signals from the sensors to other circuits or to external devices, or a processor for gathering and processing data from sensors and transmitting the data through a wire 19. The concentrator 115 receives signal from the acceleration sensor 116 mounted together with the concentrator 115 on the PCB of the main module 110. The acceleration sensor 116 can be a 3-axis MEMS sensor. Moreover, the force-sensitive layer 183 is connected to the concentrator via appropriate amplifying circuits, separated from the measurement module 20 by resilient contacts, and separated from the tool via the rubber cover 185. During operation of the system, the acceleration sensor 116 provides information related to amplitude of vibrations and the force-sensitive layer 183 provides information related to the force by which the vibrations act on the bottom part 12 of the casing, and thereby to the body of the operator.

In the second embodiment, force measurement is performed within the bottom area of the replaceable base 12, which does not require the need for calibration of force measurement after the base 12 is replaced, as the information about sensitivity of the force-sensitive layer 183 can be stored in the TEDS memory on the elastic PCB 182 integrated with the replaceable base 12.

## Claims

1. An integrated vibrations and contact force converter, comprising:
- a first element (11) separated from a second element (12) by an elastic spacer (170);
- at least one acceleration sensor (116) rigidly connected to the first element (11) and configured to measure amplitude of acceleration of vibrations of the converter;
- at least one contact force sensitive element (151, 152, 183) rigidly connected either to the first element (11) or to the second element (12) and configured to measure a force exerted on the second element (12);
**characterized in that**:
- the first element (11) and the second element (12) are connected by a connector (16) configured to maintain the second element (12) movable with respect to the first element (11) and to provide a non-zero calibrated pressing force from the first element (11) to the second element (12) at the absence of forces external to the converter.

2. The integrated converter according to claim 1, comprising a pair of force sensors (151, 152) mounted between the first element (11) and the second element (12) and configured to measure the force exerted by the first element (11) on the second element (12).

3. The integrated converter according to claim 2, wherein the acceleration sensor (116) and the force sensors (151, 152) are mounted on a common measurement module (20) which is permanently connected with the first element (11) and the second element (12) is replaceable with respect to the first element.

4. The integrated converter according to any of previous claims, wherein the at least one contact force sensitive element (151, 152) is connected movably via the elastic spacer (170) with the second element (12).

5. The integrated converter according to claim 1, wherein the contact force - sensitive element is a contact force sensitive layer (183) arranged on the area of the second element (12).

6. The integrated converter according to claim 5, wherein the acceleration sensor (116) is mounted on a common measurement module (20), which is permanently connected with the first element (11) and to which resilient contact elements (192) are connected, having ends which contact an elastic PCB (182) for conducting signals from the contact force sensitive layer (183).

7. The integrated converter according to any of previous claims, wherein the first element (11) and the second element (12) form a casing (10) of the integrated converter.

8. The integrated converter according to any of previous claims, wherein the elastic spacer (170) has at least 80 Shore A hardness.

9. The integrated converter according to any of previous claims, wherein the contact force sensitive elements (151, 152, 183) are force sensitive resistors.

10. The integrated converter according to any of previous claims, comprising a strap (17) for attaching the converter to the internal side of the palm.

11. The integrated converter according to any of previous claims, wherein the whole volume between the measurement module (20) and the top part (11) of the casing is filled with a stiffening agent, preferably epoxy resin.

12. A measurement system comprising the integrated converter according to any of claims 1 to 10 and a set of replaceable second elements (12) having different shapes.

13. A method for measuring vibrations and contact force transmitted from a vibrating tool to operator's body by an integrated converter according to any of claims from 1 to 10, comprising the steps of:
- positioning the converter between the vibrating tool and the operator's body;
- and next reading an amplitude of vibrations output by the acceleration sensor (116) and a force output by the contact force sensitive element (151, 152, 183).

## Patentansprüche

1. Integrierter Schwingungs- und Kontaktkraftwandler, umfassend:
- ein erstes Element (11), das von einem zweiten Element (12) durch einen elastischen Abstandshalter (170) getrennt ist;
- mindestens einen Beschleunigungssensor (116), der fest mit dem ersten Element (11) verbunden und konfiguriert ist, um Beschleunigungsamplitude von Schwingungen des Wandlers zu messen;
- mindestens ein kontaktkraftempfindliches Element (151, 152, 183), das fest entweder mit dem ersten Element (11) oder mit dem zweiten Element (12) verbunden und konfiguriert ist, um eine Kraft zu messen, die auf das zweite Element (12) ausgeübt wird;
**dadurch gekennzeichnet, dass**:
- das erste Element (11) und das zweite Element (12) durch einen Verbinder (16) verbunden sind, der konfiguriert ist, um das zweite Element (12) in Bezug auf das erste Element (11) beweglich zu halten und um eine kalibrierte Druckkraft ungleich null von dem ersten Element (11) auf das zweite Element (12) bei der Abwesenheit von Kräften außerhalb des Wandlers bereitzustellen.

2. Integrierter Wandler nach Anspruch 1, umfassend ein Paar Kraftsensoren (151, 152), die zwischen dem ersten Element (11) und dem zweiten Element (12) montiert und konfiguriert sind, um die Kraft zu messen, die durch das erste Element (11) auf das zweite Element (12) ausgeübt wird.

3. Integrierter Wandler nach Anspruch 2, wobei der Beschleunigungssensor (116) und die Kraftsensoren (151, 152) auf einem gemeinsamen Messmodul (20) montiert sind, das permanent mit dem ersten Element (11) verbunden ist und das zweite Element (12) in Bezug auf das erste Element austauschbar ist.

4. Integrierter Wandler nach einem der vorhergehenden Ansprüche, wobei das mindestens eine kontaktkraftempfindliche Element (151, 152) über den elastischen Abstandshalter (170) beweglich mit dem zweiten Element (12) verbunden ist.

5. Integrierter Wandler nach Anspruch 1, wobei das kontaktkraftempfindliche Element eine kontaktkraftempfindliche Schicht (183) ist, die auf dem Bereich des zweiten Elements (12) angeordnet ist.

6. Integrierter Wandler nach Anspruch 5, wobei der Beschleunigungssensor (116) auf einem gemeinsamen Messmodul (20) montiert ist, das permanent mit dem ersten Element (11) verbunden ist und mit dem federnde Kontaktelemente (192) verbunden sind, die Enden aufweisen, die eine elastische Leiterplatte (182) kontaktieren, um Signale von der kontaktkraftempfindlichen Schicht (183) zu leiten.

7. Integrierter Wandler nach einem der vorhergehenden Ansprüche, wobei das erste Element (11) und das zweite Element (12) ein Gehäuse (10) des integrierten Wandlers bilden.

8. Integrierter Wandler nach einem der vorhergehenden Ansprüche, wobei der elastische Abstandshalter (170) eine Härte von mindestens 80 Shore A aufweist.

9. Integrierter Wandler nach einem der vorhergehenden Ansprüche, wobei die kontaktkraftempfindlichen Elemente (151, 152, 183) kraftempfindliche Widerstände sind.

10. Integrierter Wandler nach einem der vorhergehenden Ansprüche, umfassend ein Band (17) zum Anbringen des Wandlers an der Innenseite der Handfläche.

11. Integrierter Wandler nach einem der vorhergehenden Ansprüche, wobei das gesamte Volumen zwischen dem Messmodul (20) und dem oberen Teil (11) des Gehäuses mit einem Versteifungsmittel, bevorzugt Epoxidharz, gefüllt ist.

12. Messsystem, das den integrierten Wandler nach einem der Ansprüche 1 bis 10 und einen Satz austauschbarer zweiter Elemente (12) mit unterschiedlichen Formen umfasst.

13. Verfahren zum Messen von Schwingungen und Kontaktkraft, die von einem Schwingungswerkzeug auf Körper eines Bedieners übertragen werden, durch einen integrierten Wandler nach einem der Ansprüche 1 bis 10, umfassend die folgenden Schritte:
- Positionieren des Wandlers zwischen dem Schwingungswerkzeug und dem Körper des Bedieners;
- und als nächstes Lesen einer Schwingungsamplitude, die durch den Beschleunigungssensor (116) ausgegeben wird, und einer Kraft, die durch das kontaktkraftempfindliche Element (151, 152, 183) ausgegeben wird.

## Revendications

1. Convertisseur intégré de vibrations et de force de contact, comprenant :
- un premier élément (11) séparé d'un second élément (12) par une entretoise élastique (170) ;
- au moins un capteur d'accélération (116) relié rigidement au premier élément (11) et conçu pour mesurer l'amplitude d'accélération des vibrations du convertisseur ;
- au moins un élément sensible à la force de contact (151, 152, 183) relié rigidement soit au premier élément (11) soit au second élément (12) et conçu pour mesurer une force exercée sur le second élément (12) ;
**caractérisé en ce que** :
- le premier élément (11) et le second élément (12) sont reliés par un connecteur (16) conçu pour maintenir le second élément (12) mobile par rapport au premier élément (11) et pour fournir une force de pression étalonnée non nulle du premier élément (11) sur le second élément (12) en l'absence de forces extérieures sur le convertisseur.

2. Convertisseur intégré selon la revendication 1, comprenant une paire de capteurs de force (151, 152) montés entre le premier élément (11) et le second élément (12) et conçus pour mesurer la force exercée par le premier élément (11) sur le second élément (12).

3. Convertisseur intégré selon la revendication 2, ledit capteur d'accélération (116) et lesdits capteurs de force (151, 152) étant montés sur un module de mesure commun (20) qui est relié en permanence au premier élément (11) et ledit second élément (12) étant remplaçable par rapport au premier élément.

4. Convertisseur intégré selon l'une quelconque des revendications précédentes, ledit au moins un élément sensible à la force de contact (151, 152) étant relié de manière mobile par l'intermédiaire de l'entretoise élastique (170) au second élément (12).

5. Convertisseur intégré selon la revendication 1, ledit élément sensible à la force de contact étant une couche sensible à la force de contact (183) agencée sur la zone du second élément (12).

6. Convertisseur intégré selon la revendication 5, ledit capteur d'accélération (116) étant monté sur un module de mesure commun (20), qui est relié en permanence au premier élément (11) et auquel sont reliés des éléments de contact élastiques (192), possédant des extrémités qui entrent en contact avec une PCB élastique (182) pour conduire des signaux en provenance de la couche sensible à la force de contact (183).

7. Convertisseur intégré selon l'une quelconque des revendications précédentes, ledit premier élément (11) et ledit second élément (12) formant un boîtier (10) du convertisseur intégré.

8. Convertisseur intégré selon l'une quelconque des revendications précédentes, ladite entretoise élastique (170) possédant une dureté supérieure ou égale à 80 Shore A.

9. Convertisseur intégré selon l'une quelconque des revendications précédentes, lesdits éléments sensibles à la force de contact (151, 152, 183) étant des résistances sensibles à la force.

10. Convertisseur intégré selon l'une quelconque des revendications précédentes, comprenant une sangle (17) destinée à attacher le convertisseur au côté interne de la paume.

11. Convertisseur intégré selon l'une quelconque des revendications précédentes, tout le volume entre le module de mesure (20) et la partie supérieure (11) du boîtier étant rempli d'un agent raidissant, de préférence une résine époxy.

12. Système de mesure comprenant le convertisseur intégré selon l'une quelconque des revendications 1 à 10 et un ensemble de seconds éléments remplaçables (12) possédant des formes différentes.

13. Procédé permettant la mesure des vibrations et de la force de contact transmises d'un outil vibrant au corps de l'opérateur par un convertisseur intégré selon l'une quelconque des revendications 1 à 10, comprenant les étapes de :
- positionnement du convertisseur entre l'outil vibrant et le corps de l'opérateur ;
- et ensuite lecture d'une amplitude de vibrations délivrée en sortie par le capteur d'accélération (116) et d'une force délivrée en sortie par l'élément sensible à la force de contact (151, 152, 183).
